# EUROPEAN PATENT SPECIFICATION

(11) **EP 0 421 469 B1**
(45) Date of publication and mention of the grant of the patent: **15.04.1998**
(21) Application number: 90119175.9
(22) Date of filing: 05.10.1990
(51) Int. Cl.: C12Q 1/68, C07H 21/04

(54) **Method for separating a target oligonucleotide**
Verfahren zur Trennung eines Zieloligonucleotids
Procédé pour la séparation d'un oligonucléotide cible

(30) Priority: 06.10.1989 JP 261298/89
(43) Date of publication of application: 10.04.1991
(73) Proprietor: CANON KABUSHIKI KAISHA, Tokyo (JP)
(72) Inventor: Kato, Kinya, c/o Canon Kabushiki Kaisha, Ohta-ku, Tokyo (JP); Iwashita, Harumi, c/o Canon Kabushiki Kaisha, Ohta-ku, Tokyo (JP); Yamamoto, Nobuko, c/o Canon Kabushiki Kaisha, Ohta-ku, Tokyo (JP); Sakuranaga, Masanori, c/o Canon Kabushiki Kaisha, Ohta-ku, Tokyo (JP)
(74) Representative: Bühling, Gerhard, Dipl.-Chem.

(56) References cited:
- EP-A- 0 167 238
- EP-A- 0 194 545
- EP-A- 0 225 807
- EP-A- 0 317 074
- EP-A- 0 332 435
- WO-A-89/06285
- WO-A-90/06374
- WO-A-90/09455
- US-A- 4 851 331
- NUCLEIC ACIDS RESEARCH vol. 16, no. 23, September 1988, OXFORD, GB. pages 11327 - 11338 SYV[NEN, A-C ET AL. 'Quantification of polymerase products by affinity- based hybrid collection.'

## Description

### BACKGROUND OF THE INVENTION

### Field of the Invention

The present invention relates to a method for separating a nucleic acid which is useful for detecting or determining nucleic acids of viruses, microorganisms, vegetables, animals, or the like.

### Related Background Art

In hybridization reaction, a labelled oligonucleotide or a labelled polynucleotide, namely a probe, is capable of forming a base pair with a target nucleic acid.

For detection of nucleic acids, a variety of hybridization methods have been employed. In a direct hydridization method, the specimen is dissolved in a solution or immobilized by a solid carrier. The nucleic acid to be immobilized is demonstrated by use of a labelled probe.

US Patent 4,486,539 describes a sandwich hybridization method, in which two separate probes are used: the one is a detection probe which is labelled and utilized for detection, and the other is a capture probe immobilized on a solid carrier for separating a target nucleic acid from the reaction mixture.

Such conventional hybridization analysis of nucleic acids employing a filter as a carrier involves a problem that much time is required for a series of operation steps of immobilization of the nucleic acid in a specimen, hybrid formation with a target probe, washing, and detection, and is not suitable for routine clinical experiment.

In particular, the operations in immobilization of a nucleic acid, such as fixing, prehybridization, and washing are complicated, requiring much time for analysis [Lin, et al.: Journal of Virology, Vol. 55, p. 509 (1985)]

Further the hybrid formation on the filter involves a problem that the rate of hybrid formation is low because the reaction of a single stranded nucleic acid in a solid phase with a labelled probe in a liquid is made to proceed in a non-uniform state in a solution. Moreover, the hybrid is formed in such a low efficiency that usually only several percent of the calculated expected quantity of the added labelled probe is utilized for the hybrid formation.

Furthermore, the hybrid formation analysis of a nucleic acid employing a filter as a carrier involves a problem of low sensitivity and low accuracy.

Accordingly, hybridization in a solution is being investigated lately.

A hybridization method in solution is described in British Patent Laid-open Publication No. 2,169,403, which employs two different probes coexisting in the same liquid phase. The detection probe is labelled by a detectable label, while the capture probe has a portion having affinity to another moiety. After hybridization, the hybrid formed from the capture probe, the target nucleic acid, and the detection probe can be separated by the other portion of the affinity pair from the hybridization solution. The method mentioned above employs two probes, namely a capture probe and a detection probe, so that the two probes are required to be prepared and to be treated for capturing and labelling respectively.

On the other hand, although it may be considered that one portion of the probe is treated for the capture and another portion of the same probe is treated for labelling, the treatments have to be conducted separately and are naturally complicated.

The EP-A-0 348 529 discloses a method for detecting a target nucleic acid, wherein a probe nucleic acid complementary to a sample or target nucleic acid is hybridized with said sample nucleic acid. During the following hybridization and extension reaction, the probe nucleic acid acts as a primer such that the primer itself, after being bound to the target, is provided in the form of a single-stranded DNA molecule.

Additionally, in conventional methods, probes are modified for the capture, which adversely affects hybridization resulting in lack of precision. Since the modification for the capture is directed to relatively long probes, it is not suitable for short probes, which makes difficult the detection of point mutation.

### SUMMARY OF THE INVENTION

An object of the present invention is to provide a method for separating a nucleic acid, which does not have disadvantages of the prior art, and is utterly simple and applicable to high-sensitive detection or determination of nucleic acids.

According to an aspect of the present invention, there is provided a method for separating a target oligonucleotide according to claim 1.

According to another aspect of the present invention there is provided a method for detecting a target oligonucleotide according to either claim 6 or claim 7.

### BRIEF DESCRIPTION OF THE DRAWINGS

Figs. 1A to 1C are schematic diagrams showing the method of the present invention.

Figs. 2A to 2F illustrate an example of a separation process (a fractionation process) of the present invention.

Figs. 3A to 3F illustrate another example of the separation process (a fractionation process) of the present invention.

Figs. 4A to 4C are schematic diagrams showing another method of the present invention.

### DETAILED DESCRIPTION OF THE PREFERRED EMBODIMENT

The sequences of DNA or RNA to be detected according to the present invention are not limited to those of specified living bodies, but include those of viruses, microorganisms such as bacteria, eumycetes, and protistas, vegetables and animals regardless of whether the DNA or RNA is a fragment, a double-stranded one, or a single-stranded one.

The base sequence of the probe is complementary to the DNA or RNA base sequence to be detected for the presence or the absence of the specified nucleic acid sequence. The number of the bases is not limited, but is selected optimally depending on the DNA or RNA to be detected.

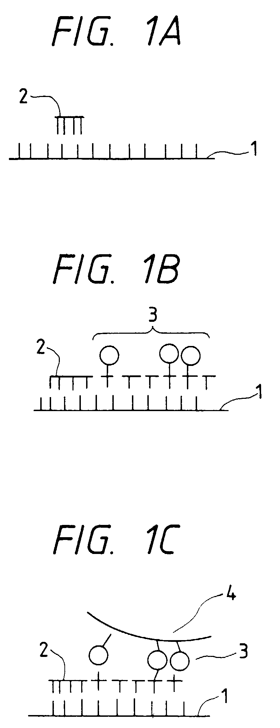

Figs. 1A, 1B, and 1C show schematic diagrams illustrating a method of the present invention. In the Figs. the numeral 1 denotes a target nucleic acid; 2, a probe nucleic acid; 3, an immobilizing substance; and 4, a carrier.

Fig. 1A and Fig. 1B illustrate introduction of an immobilizing substance into a hybrid former by extension reaction.

Thereafter, as shown in Fig. 1C, a carrier is bonded to the immobilizing substance. The immobilizing substance in the present invention is a nucleotide triphosphate derivative prepared by introducing a bonding group capable of forming a bond, with specific affinity for a carrier, into a nucleotide triphosphate to be incorporated in a DNA hybrid former by an extension reaction in the presence of a polymerizing agent. Specifically, the nucleotide triphosphate derivative includes biotin, heavy metal derivatives, homopolynucleotides, and the like.

The aforementioned polymerizing agent includes E. coli DNA polymerase, a Klenow fragment of E. coli DNA polymerase I, T4 DNA polymerase, T7 DNA polymerase, thermostable DNA polymerase, a reverse transcriptase, and the like.

The carrier may be any material in the present invention, if the material has the properties of forming a bond by affinity with the aforementioned nucleotide triphosphate derivative, and not forming a bond with the nucleotide triphosphate to separate the hybrid former from the non-hybrid former by bonding to the nucleotide triphosphate derivative.

Specifically, the affinity pair portion is a component having affinity with another component. Examples of such affinity pair are biotin-avidin or - streptoavidin, a heavy metal-a thio group, a variety of homopolynucleotides such as polydG-polydC, polydA-polydT, polydA-polydU, and the like. In the present invention, the carriers are prepared by bonding the aforementioned avidin, streptoavidin, a thio group, or a homopolynucleotide to a surface of a gel.

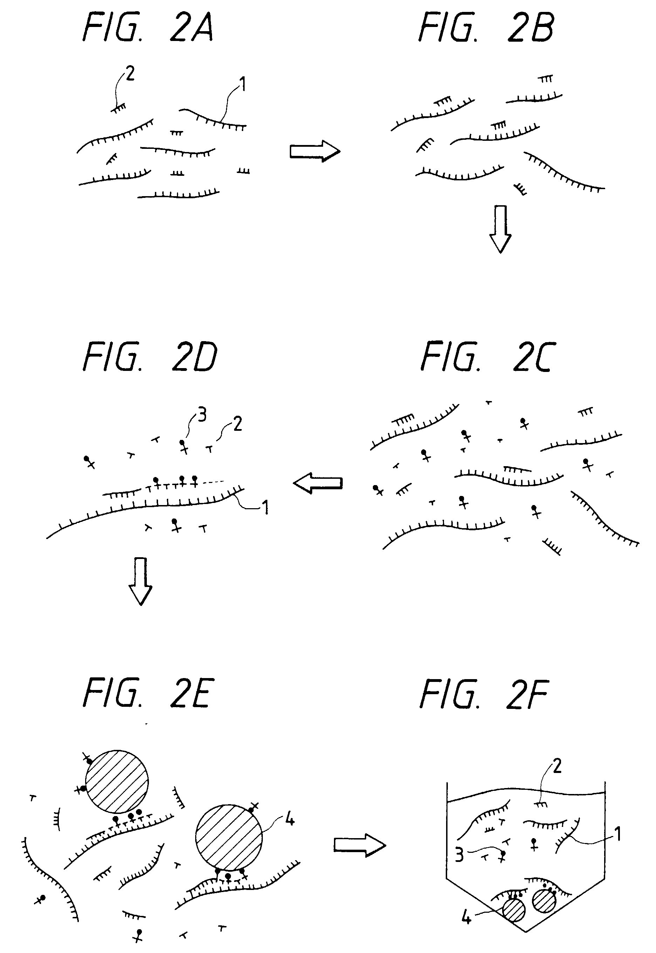

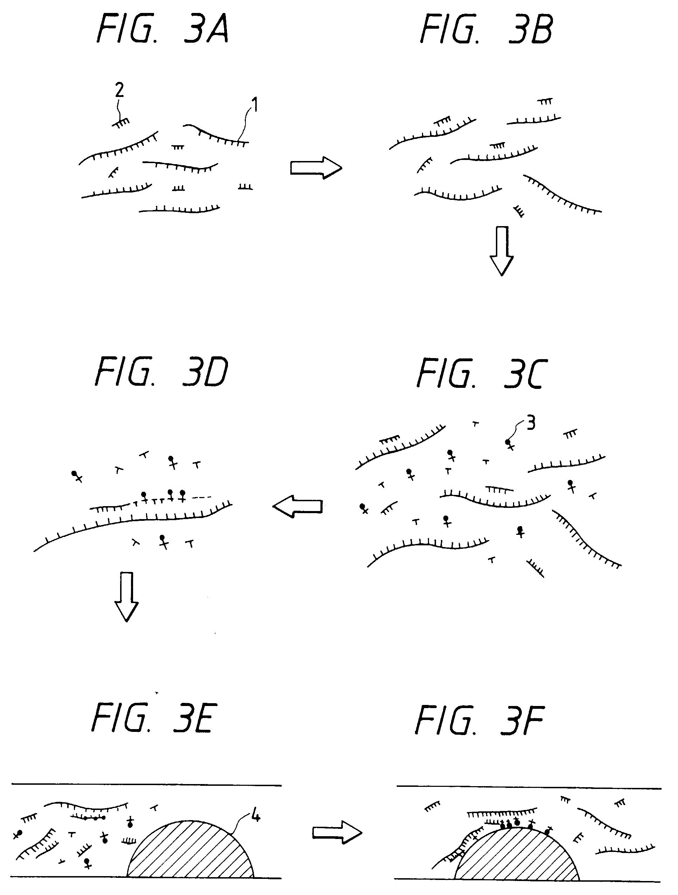

Fig. 2 and Fig. 3 illustrate other embodiment of the separation of the present invention.

In the present invention, a labelling substance may be preliminarily introduced into the probe nucleic acid in the method. Further, the labelling need not be preliminarily conducted, so that the process is more simplified. The method comprises the steps of introducing an immobilizing substance, which reacts specifically with a carrier and a labelling substance, only into the hybrid former by extension reaction; and separating the hybrid former having the immobilizing substance and the labelling substance by bonding the immobilized substance with a carrier from a non-hybrid former in the mixture of the reaction solution.

The labelling substance includes radioactive isotopes, fluorescent substances, chemiluminescent substances, enzymes, and the like.

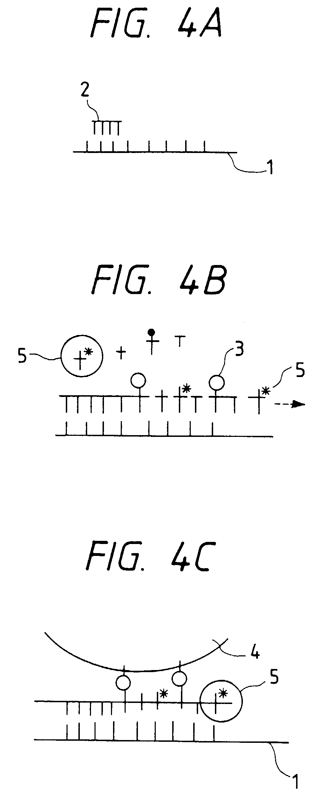

In Fig. 4, the numerals 1 to 4 denote the same as in the preceding figures. The numeral 5 denotes a labelling substance.

The embodiment of the present invention is described more specifically with examples.

### Example 1

Nucleic acid detection was conducted to examine whether a nucleic acid sample has a partial base sequence of plasmid pUC19.

As a nucleic acid for the probe, two oligonucleotides shown below which correspond to a partial base sequence of plasmid pUC19 were synthesized by means of a DNA synthesis apparatus (Model 381A, made by ABI Co.) 
5'GATCGCCCTTCCCAACAGTTGCGCAG3'
5'CATTCGCCATTCAGGCTGCGCAA3'
 In the above sequence, 8 bases from 3' terminals are complementary to each other.

Portions of the synthesized nucleotides were tested for the purity by electrophoresis employing 20% polyacrylamide containing 7M urea. The purity was found to be not less than 95 % respectively. Accordingly, the oligonucleotides were used without further purification in the following reactions.

In a Epfendorf tube, 2 µg (approximately 130 pmoles) each of the oligonucleotides, 5 µl of a 10x annealing solution (100mM Tris - HCl pH8.0 - 60mM MgCl₂ - 60mM β-mercaptoethanol - 500mM NaCl) were added, and distilled water was added to the total volume of 50 µl.

The tube was heated for 10 minutes in a beaker containing hot water kept at 65°C, and then gradually cooled to room temperature (over approximately one hour). This reaction causes the formation of a partially double-stranded chain of the two oligonucleotides as shown below:

5'GATCGCCCTTCCCAACAGTTGCGCAG
AACGCGTCGGACTTACCGCTTAC5'

To 20 µl of this annealed oligonucleotide solution, 2 µl of a 10x annealing solution, 2 µl each of 1 mM dATP, dCTP, and dGTP, and further 100µCi of P-TTP were added. Water was added thereto to the total volume of 40 µl. One unit of Klenow fragment of DNA polymerase I was added and the reaction was allowed to proceed for one hour in ice.

The reaction mixture was extracted with phenol, and ethanol was added thereto to form a precipitate. The precipitate was dissolved in 20 µl of ddH₂O to prepare a probe solution.

On the other hand, three samples: pBR322, pUC19, and a mixture thereof, were provided as the nucleic acids to be detected. Hereinafter, the samples pBR322, pUC19, and the mixture of pBR322 with pUC19 are referred to as Sample, A, Sample B, and Sample C, respectively.

The samples A, B, and C were treated in the same manner. To 1 µg/2 µl of the sample DNA, 2.0 µl of 10x TA buffer solution, and 16 µl ddH₂O were added. Thereto 10 units of Hind III was added, and the sample was completely decomposed at 37°C for 2 hours. The reaction mixture was extracted with phenol, and ethanol was added to cause precipitate formation. The precipitate was dissolved in 20 µl of ddH₂O. Thereto 20 µl of the probe solution prepared before (having been heated at 90°C for 5 minutes) and 4 µl of a 10x annealing solution (100mM Tris - HCl pH8.0 - 60mM MgCl₂ - 60mM β-mercaptoethanol - 500mM NaCl). The solution was heated to 65°C, kept at that temperature for 10 minutes, and cooled gradually to room temperature (over 3 hours). Then the solution was extracted with phenol, and ethanol was added thereto to form precipitate. The precipitate was dissolved in ddH₂O.

To this solution, 2 µl each of 1mM dATP, dGTP, and dCTP, 5 µl of 0.4 mM biotinylated UTP (made by BRL Co.), 5 µl of a 10x annealing solution, and 32 µl of distilled water were added, and mixed sufficiently. 16 units of a Klenow fragment of DNA polymerase I (made by Toyobo Co.) was added thereto, and the mixture was heated at 37°C for one hour to cause extension reaction.

The gel particles (of approximately 5 mm in diameter), which were prepared by crushing agarose gel, activating the surface thereof with cyan bromide, and bonding it to avidin, were added to the solution. The gel particles were blended with the solution for 20 minutes. In this step, the biotin incorporated into DNA is specifically bonded to the avidin on the gel particle surface.

Subsequently, the mixture was centrifuged gently, and the supernatant was discarded. Having been washed with TE twice, the precipitate was subjected to scintillation counting measurement. The biological sample containing pUC19 had an intensity of not less tha 10⁶⁻⁷ cpm, while the biological sample containing pBR322 had an intensity of below twice that of the background. Thus the difference between the two samples is obvious.

### Example 2

Nucleic acid detection was conducted to examine whether a nucleic acid sample has a partial base sequence of plasmid pUC19.

As a nucleic acid for the probe, an oligonucleotide shown below which corresponds to a partial base sequence of plasmid pUC19 were synthesized by means of a DNA synthesis apparatus (Model 381A, made by ABI Co.)

5'GATCGCCCTTCCCAACAGTTGCGCAGCCTGAATGGCGAATG

Portions of the synthesized nucleotides were tested for the purity by electrophoresis employing 20% polyacrylamide containing 7M urea. The purity was found to be not less than 95 % respectively. Accordingly, the oligonucleotides were used without further purification in the following reactions.

On the other hand, three samples: pBR322, pUC19, and a mixture thereof, were provided as the nucleic acids to be detected. Hereinafter, the samples pBR322, pUC19, and the mixture of pBR322 with pUC19 are referred to as Sample, A, Sample B, and Sample C, respectively.

The samples A, B, and C were treated in the same manner. To 1 µg/2 µl of the sample DNA, 2.0 µl of 10x TA buffer solution, and 16 µl ddH₂O were added. Thereto 10 units of Hind III was added, and the sample was completely decomposed at 37°C for 2 hours. The reaction mixture was extracted with phenol, and ethanol was added to cause precipitate formation. The precipitate was dissolved in 20 µl of ddH₂O. Thereto 20 µl of the probe solution prepared before (having been heated at 90°C for 5 minutes) and 4 µl of a 10x annealing solution (100mM Tris - HCl pH8.0 - 60mM MgCl₂ - 60mM β-mercaptoethanol - 500mM NaCl). The solution was heated to 65°C, kept at that temperature for 10 minutes, and cooled gradually to room temperature (over 3 hours). Then the solution was extracted with phenol, and ethanol was added thereto to form precipitate. The precipitate was dissolved in ddH₂O.

To this solution, 2 µl each of P-TTP100µCi, 1mM dATP, dGTP, and dCTP, 5 µl of 0.4 mM biotinylated UTP (made by BRL Co.), 5 µl of a 10x annealing solution, and 32 µl of distilled water were added, and mixed sufficiently. 16 units of a Klenow fragment of DNA polymerase I (made by Toyobo Co.) was added thereto, and the mixture was heated at 37°C for one hour to cause extension reaction.

The gel particles (of approximately 5 mm in diameter), which were prepared by crushing agarose gel, activating the surface thereof with cyan bromide, and bonding it to avidin, were added to the solution. The gel particles were blended with the solution for 20 minutes. In this step, the biotin incorporated into DNA is specifically bonded to the avidin on the gel particle surface.

Subsequently, the mixture was centrifuged gently, and the supernatant was discarded. Having been washed with TE twice, the precipitate was subjected to scintillation counting measurement. The biological sample containing pUC19 had an intensity of not less tha 10⁶⁻⁷ cpm, while the biological sample containing pBR322 had an intensity of below twice that of the background. Thus the difference between the two samples is obvious.

As discussed above, the present invention makes it feasible to incorporate both a capture substance and a labelling substance into a hybridized matter, while in the conventional method a capture probe and a labelling probe have to te prepared separately. Thus the present invention eliminates complicated processes and the need for providing two kinds of probes.

Further in the present invention, use of unmodified probe eliminates the influence of the modification (namely, unpreciseness) on hybridization.

The present invention enables immobilization after hybridization, allowing the hybridization to proceed without any restriction of both a probe and a target DNA, improving considerably the efficiency of the hybridization, and suppressing the background. Since specific immobilization on an immobilizing phase is made possible, the degree of freedom of immobilization states and sites is increased, thus making feasible a variety of detection in gene diagnosis.

## Claims

1. A method for separating a target oligonucleotide, comprising:
a) preparing a probe oligonucleotide by the steps of:
i) synthesizing at least one pair of oligonucleotides capable of binding with each other through complementary sequences at the 3'-end regions of the oligonucleotides,
ii) binding the at least one pair of oligonucleotides with each other via the complementary sequences to form a partially double-stranded oligonucleotide, and then carrying out an extension reaction to polymerize nucleotides onto the partially double-stranded oligonucleotide,
iii) denaturing the double-stranded oligonucleotide to form single-stranded probe oligonucleotides;
b) reacting the single-stranded probe oligonucleotides with a target single-stranded oligonucleotide to form a double-stranded hybrid;
c) carrying out an extension reaction in a reaction solution containing a nucleotide to which an immobilizing substance is bound, thereby polymerizing oligonucleotides onto the double-stranded hybrid and incorporating the immobilizing substance into the extended part of the double-stranded hybrid obtained by the extension reaction;
d) binding the immobilizing substance incorporated into the double-stranded hybrid to a carrier, which is specifically reactive with the immobilizing substance; and
e) separating the hybrid bound to the carrier from an unhybridized oligonucleotide in a reaction solution.

2. The method of separating a nucleic acid of Claim 1, wherein the probe nucleic acid has been labelled.

3. The method of separating a nucleic acid of Claim 1, wherein the immobilized substance is selected from biotin, heavy metal derivatives, and homopolynucleic acids.

4. The method of separating a nucleic acid of Claim 1, wherein the carrier has avidin, streptoavidin, a thio group, or a homopolynucleic acid.

5. The method of separating a nucleic acid of Claim 1, wherein the carrier is formed by bonding avidin, streptavidin, a thio group, or a homopolynucleotide onto a surface of gel particles.

6. A method for detecting a target oligonucleotide, comprising:
a) preparing a labeled probe oligonucleotide by the steps of:
i) synthesizing at least one pair of oligonucleotides capable of binding with each other through complementary sequences at 3'-end regions of the oligonucleotides,
ii) binding the at least one pair of oligonucleotides with each other via the complementary sequences to form a partially double-stranded oligonucleotide, and then carrying out an extension reaction in a reaction solution containing a nucleotide to which a labeling substance is bound, thereby polymerizing nucleotides onto the partially double-stranded oligonucleotide and incorporating the labeling substance into the extended part of the double-stranded hybrid obtained by the extension reaction,
iii) denaturing the double-stranded oligonucleotide to form single-stranded labeled probe oligonucleotides;
b) reacting the single-stranded labeled probe oligonucleotides with a target single-stranded oligonucleotide to form a double-stranded labeled hybrid;
c) carrying out an extension reaction in a reaction solution containing a nucleotide to which an immobilizing substance is bound, thereby polymerizing oligonucleotides onto the double-stranded labeled hybrid and incorporating the immobilizing substance into the extended part of the double-stranded labeled hybrid obtained by the extension reaction;
d) binding the immobilizing substance incorporated into the double-stranded labeled hybrid to a carrier, which is specifically reactive with the immobilizing substance;
e) separating the hybrid bound to the carrier from an unhybridized oligonucleotide in a reaction solution; and
f) detecting the separated hybrid by the use of the labeling substance incorporated into the hybrid.

7. A method for detecting a target oligonucleotide, comprising:
a) preparing a probe oligonucleotide by the steps of:
i) synthesizing at least one pair of oligonucleotides capable of binding with each other through complementary sequences at 3'-end regions of the oligonucleotides,
ii) binding the at least one pair of oligonucleotides with each other via the complementary sequences to form a partially double-stranded oligonucleotide, and then carrying out an extension reaction to polymerize nucleotides onto the partially double-stranded oligonucleotide,
iii) denaturing the double-stranded oligonucleotide to form single-stranded probe oligonucleotides;
b) reacting the single-stranded probe oligonucleotides with a target single-stranded oligonucleotide to form a double-stranded hybrid;
c) carrying out an extension reaction in a reaction solution containing a nucleotide to which an immobilizing substance is bound and a nucleotide to which a labeling substance is bound, thereby polymerizing oligonucleotides onto the double-stranded hybrid and incorporating the immobilizing substance and the labeling substance into the extended part of the double-stranded hybrid obtained by the extension reaction;
d) binding the immobilizing substance incorporated into the double-stranded hybrid to a carrier, which is specifically reactive with the immobilizing substance;
e) separating the hybrid bound to the carrier from an unhybridized oligonucleotide in a reaction solution; and
f) detecting the separated hybrid by the use of the labeling substance incorporated into the hybrid.

## Patentansprüche

1. Verfahren zur Trennung eines Ziel-Oligonukleotids, umfassend:
a) Herstellung einer Oligonukleotid-Probe durch die Schritte:
i) Synthese mindestens eines Paars von Oligonukleotiden, die aneinander durch komplementäre Sequenzen an den 3'-Endbereichen der Oligonukleotide binden können,
ii) Aneinanderbinden des mindestens einen Paars von Oligonukleotiden über die komplementären Sequenzen unter Bildung eines teilweise doppelsträngigen Oligonukleotids, und darauffolgende Durchführung einer Verlängerungsreaktion zur Polymerisation von Nukleotiden an dem teilweise doppelsträngigen Oligonukleotid,
iii) Denaturierung des doppelsträngigen Oligonukleotids unter Bildung einsträngiger Oligonukleotid-Proben;
b) Umsetzung der einsträngigen Oligonukleotid-Proben mit einem einsträngigen Ziel-Oligonukleotid unter Bildung eines doppelsträngigen Hybrids;
c) Durchführung einer Verlängerungsreaktion in einer Reaktionslösung, in welcher ein mit einer immobilisierenden Substanz verbundenes Nukleotid enthalten ist, wobei dadurch Oligonukleotide an dem doppelsträngigen Hybrid polymerisiert werden und die immobilisierende Substanz in den durch die Verlängerungsreaktion erhaltenen verlängerten Teil des doppelsträngigen Hybrids eingefügt wird;
d) Bindung der in das doppelsträngige Hybrid eingefügten immobilisierenden Substanz an einen Träger, der spezifisch mit der immobilisierenden Substanz reagiert; und
e) Trennung des an den Träger gebundenen Hybrids von einem nicht hybridisierten Oligonukleotid in einer Reaktionslösung.

2. Verfahren zur Trennung einer Nukleinsäure nach Anspruch 1, wobei die Nukleinsäure-Probe markiert wurde.

3. Verfahren zur Trennung einer Nukleinsäure nach Anspruch 1, wobei die immobilisierende Substanz aus Biotin, Schwermetall-Derivaten und Homopolynukleinsäuren ausgewählt ist.

4. Verfahren zur Trennung einer Nukleinsäure nach Anspruch 1, wobei der Träger Avidin, Streptoavidin, eine Thiogruppe oder eine Homopolynukleinsäure aufweist.

5. Verfahren zur Trennung einer Nukleinsäure nach Anspruch 1, wobei der Träger durch Bindung von Avidin, Streptoavidin, einer Thiogruppe oder eines Homopolynukleotids an eine Oberfläche von Gel-Partikeln gebildet wird.

6. Verfahren zur Erfassung eines Ziel-Oligonukleotids, umfassend:
a) Herstellung einer markierten Oligonukleotid-Probe durch die Schritte:
i) Synthese mindestens eines Paars von Oligonukleotiden, die aneinander durch komplementäre Sequenzen an den 3'-Endbereichen der Oligonukleotide binden können,
ii) Aneinanderbinden des mindestens einen Paars von Oligonukleotiden über die komplementären Sequenzen unter Bildung eines teilweise doppelsträngigen Oligonukleotids, und darauffolgende Durchführung einer Verlängerungsreaktion in einer Reaktionslösung, in der ein mit einer Markierungssubstanz verbundenes Nukleotid enthalten ist, um dadurch Nukleotide an dem teilweise doppelsträngigen Oligonukleotid zu polymerisieren und die Markierungssubstanz in den durch die Verlängerungsreaktion erhaltenen verlängerten Teil des doppelsträngigen Hybrids einzufügen,
iii) Denaturierung des doppelsträngigen Oligonukleotids unter Bildung einsträngiger Oligonukleotid-Proben;
b) Umsetzung der einsträngigen, markierten Oligonukleotid-Proben mit einem einsträngigen Ziel-Oligonukleotid unter Bildung eines doppelsträngigen markierten Hybrids;
c) Durchführung einer Verlängerungsreaktion in einer Reaktionslösung, in welcher ein mit einer immobilisierenden Substanz verbundenes Nukleotid enthalten ist, wobei dadurch Oligonukleotide an dem doppelsträngigen markierten Hybrid polymerisiert werden und die immobilisierende Substanz in den durch die Verlängerungsreaktion erhaltenen verlängerten Teil des doppelsträngigen markierten Hybrids eingefügt wird;
d) Bindung der in das doppelsträngige markierte Hybrid eingefügten immobilisierenden Substanz an einen Träger, der spezifisch mit der immobilisierenden Substanz reagiert;
e) Trennung des an den Träger gebundenen Hybrids von einem nicht hybridisierten Oligonukleotid in einer Reaktionslösung; und
f) Erfassung des abgetrennten Hybrids durch die Verwendung einer in das Hybrid eingefügten Markierungssubstanz.

7. Verfahren zur Erfassung eines Ziel-Oligonukleotids, umfassend:
a) Herstellung einer Oligonukleotid-Probe durch die Schritte:
i) Synthese mindestens eines Paars von Oligonukleotiden, die aneinander durch komplementäre Sequenzen an den 3'-Endbereichen der Oligonukleotide binden können,
ii) Aneinanderbinden des mindestens einen Paars von Oligonukleotiden über die komplementären Sequenzen unter Bildung eines teilweise doppelsträngigen Oligonukleotids, und darauffolgende Durchführung einer Verlängerungsreaktion zur Polymerisation von Nukleotiden an dem teilweise doppelsträngigen Oligonukleotid,
iii) Denaturierung des doppelsträngigen Oligonukleotids unter Bildung einsträngiger Oligonukleotid-Proben;
b) Umsetzung der einsträngigen Oligonukleotid-Proben mit einem einsträngigen Ziel-Oligonukleotid unter Bildung eines doppelsträngigen Hybrids;
c) Durchführung einer Verlängerungsreaktion in einer Reaktionslösung, in welcher ein mit einer immobilisierenden Substanz verbundenes Nukleotid sowie ein mit einer Markierungssubstanz verbundenes Nukleotid enthalten ist, wobei dadurch Oligonukleotide an dem doppelsträngigen markierten Hybrid polymerisiert werden und die immobilisierende Substanz sowie die Markierungssubstanz in den durch die Verlängerungsreaktion erhaltenen verlängerten Teil des doppelsträngigen Hybrids eingefügt wird;
d) Bindung der in das doppelsträngige markierte Hybrid eingefügten immobilisierenden Substanz an einen Träger, der spezifisch mit der immobilisierenden Substanz reagiert;
e) Trennung des an den Träger gebundenen Hybrids von einem nicht hybridisierten Oligonukleotid in einer Reaktionslösung; und
f) Erfassung des abgetrennten Hybrids durch die Verwendung einer in das Hybrid eingefügten Markierungssubstanz.

## Revendications

1. Procédé de séparation d'un oligonucléotide cible, comprenant :
a) la préparation d'un oligonucléotide d'essai par les étapes de :
i) synthèse d'au moins un couple d'oligonucléotides en mesure de se lier entre eux par des séquences complémentaires au niveau des zones d'extrémité (3') des oligonucléotides,
ii) la liaison du au moins un couple d'oligonucléotides entre eux via les séquences complémentaires pour former un oligonucléotide partiellement à double brin, et la réalisation ensuite d'une réaction d'élongation pour polymériser les nucléotides sur l'oligonucléotide partiellement à double brin,
iii) la dénaturation de l'oligonucléotide à double brin pour former des oligonucléotides d'essai à brin unique;
b) la mise en réaction des oligonucléotides d'essai à brin unique avec un oligonucléotide à brin unique cible pour former un hybride à double brin;
c) la mise en oeuvre d'une réaction d'élongation dans une solution de réaction contenant un nucléotide auquel est liée une substance d'immobilisation, polymérisant de ce fait des oligonucléotides sur l'hybride à double brin et incorporant la substance d'immobilisation dans la partie allongée de l'hybride à double brin obtenu par la réaction d'élongation;
d) la liaison de la substance d'immobilisation incorporée dans l'hybride à double brin avec un porteur, qui réagit spécifiquement à la substance d'immobilisation; et
e) la séparation de l'hybride lié au porteur depuis un oligonucléotide non hybridé dans une solution de réaction.

2. Procédé de séparation d'un acide nucléique selon la revendication 1, dans lequel l'acide nucléique d'essai a été marqué.

3. Procédé de séparation d'un acide nucléique selon la revendication 1, dans lequel la substance immobilisée est sélectionnée parmi la biotine, des dérivés de métaux lourds et des acides homopolynucléiques.

4. Procédé de séparation d'un acide nucléique selon la revendication 1, dans lequel le porteur présente de l'avidine, streptoavidine, un groupe thio ou un acide homopolynucléique.

5. Procédé de séparation d'un acide nucléique selon la revendication 1, dans lequel le porteur est formé par la liaison d'avidine, streptavidine, d'un groupe thio ou d'un homopolynucléotide sur une surface de particules de gel.

6. Procédé de détection d'un oligonucléotide cible, comprenant :
a) la préparation d'un oligonucléotide d'essai marqué par les étapes de :
i) synthétisation d'au moins un couple d'oligonucléotides en mesure de se lier entre eux par des séquences complémentaires au niveau de zones d'extrémité (3') des oligonucléotides,
ii) liaison d'au moins un couple d'oligonucléotides entre eux via les séquences complémentaires pour former un oligonucléotide partiellement à double brin, et réalisation ensuite d'une réaction d'élongation dans une solution de réaction contenant un nucléotide auquel est liée une substance de marquage, polymérisant de ce fait des nucléotides sur l'oligonucléotide partiellement à double brin et incorporant la substance de marquage dans la partie allongée de l'hybride à double brin obtenu par la réaction d'élongation,
iii) dénaturation de l'oligonucléotide à double brin pour former des oligonucléotides d'essai marqués à brin unique;
b) la mise en réaction des oligonucléotides d'essai marqués à brin unique avec un oligonucléotide à brin unique cible pour former un hybride marqué à double brin;
c) la mise en oeuvre d'une réaction d'élongation dans une solution de réaction contenant un nucléotide auquel est liée une substance d'immobilisation, polymérisant de ce fait des oligonucléotides sur l'hybride marqué à double brin et incorporant la substance d'immobilisation dans la partie prolongée de l'hybride marqué à double brin obtenu par la réaction d'élongation;
d) la liaison de la substance d'immobilisation incorporée dans l'hybride marqué à double brin avec un porteur, qui réagit spécifiquement à la substance d'immobilisation;
e) séparation de l'hybride lié au porteur depuis un oligonucléotide non hybridé dans une solution de réaction; et
f) la détection de l'hybride séparé à l'aide de la substance de marquage incorporée dans l'hybride.

7. Procédé de détection d'un oligonucléotide cible, comprenant :
a) la préparation d'un oligonucléotide d'essai marqué par les étapes de :
i) synthétisation d'au moins un couple d'oligonucléotides en mesure de se lier entre eux par des séquences complémentaires au niveau de zones d'extrémité (3') des oligonucléotides,
ii) la liaison du au moins un couple d'oligonucléotides entre eux via les séquences complémentaires pour former un oligonucléotide partiellement à double brin, et la réalisation ensuite d'une réaction d'élongation pour polymériser les nucléotides sur l'oligonucléotide partiellement à double brin,
iii) dénaturation de l'oligonucléotide à double brin pour former des oligonucléotides d'essai marqués à brin unique;
b) la mise en réaction des oligonucléotides d'essai marqués à brin unique avec un oligonucléotide à brin unique cible pour former un hybride à double brin;
c) la réalisation d'une réaction d'élongation dans une solution de réaction contenant un nucléotide auquel est liée une substance d'immobilisation et un nucléotide auquel est lié une substance de marquage, polymérisant de ce fait des oligonucléotides sur l'hybride marqué à double brin et incorporant la substance d'immobilisation dans la partie allongée de l'hybride marqué à double brin obtenu par la réaction d'élongation;
d) la liaison de la substance d'immobilisation incorporée dans l'hybride marqué à double brin avec un porteur, qui réagit spécifiquement à la substance d'immobilisation;
e) séparation de l'hybride lié au porteur depuis un oligonucléotide non hybridé dans une solution de réaction; et
f) la détection de l'hybride séparé à l'aide de la substance de marquage incorporée dans l'hybride.
